# EUROPEAN PATENT APPLICATION

(11) **EP 2 677 771 A1**
(43) Date of publication of application: **25.12.2013**
(21) Application number: 12745388.4
(22) Date of filing: 06.02.2012
(51) Int. Cl.: H04R 25/00, A61B 5/12, G10L 21/06

(54) **HEARING AID ADJUSTMENT DEVICE**

(30) Priority: 18.02.2011 JP 2011033255
(71) Applicant: Panasonic Corporation, Osaka 571-8501 (JP)
(72) Inventor: NISHIZAKI, Makoto, Osaka-shi, Osaka 540-6207 (JP); KONDO, Hiroshi, Osaka-shi, Osaka 540-6207 (JP)
(74) Representative: Eisenführ, Speiser & Partner
(86) International application number: PCT/JP2012/000789
(87) International publication number: WO 2012/111276

(57) **Abstract**

This hearing aid adjustment device is connected to a hearing aid and is used to adjust the hearing aid by using reference sounds, and comprises a memory section, an output section, an input section, a controller, and a display section. The memory section stores reference sounds to which segment information has been assigned, as well as the desired volume range for the hearing aid user. The output section outputs a reference sound from the memory section to the hearing aid. The input section inputs hearing aid processed sounds with respect to reference sounds that have undergone hearing aid processing in the hearing aid. The controller calculates the volume of segment units included in a hearing aid processed sound on the basis of segment information stored in the memory section. The display section displays in segment units whether or not the volume calculated by the controller falls within the desired volume range for the hearing aid user as stored in the memory section.

## Description

### TECHNICAL FIELD

The present invention relates to a hearing aid adjustment device used to adjust a connected hearing aid according to the hearing ability of the user and other such conditions.

### BACKGROUND ART

Hearing aid adjustment devices have been used in the past to adjust a hearing aid to match the hearing ability of the user. For example, Patent Literature 1 discloses a display device for a hearing aid adjustment device used in the course of hearing aid adjustment. Specifically, the uncomfortable level (UCL) and hearing threshold (HTL) of a hearing aid user are displayed on a display screen as a graph in which the horizontal axis is frequency (Hertz) and the vertical axis is the volume (dB) of the sound outputted from the hearing aid. This graph shows the maximum sound level (peak indicator) outputted from the hearing aid at various frequency bands, and the hearing aid user can hear a sound properly as long as the peak indicator is between the UCL and the HTL. Thus, whether or not a hearing aid has been properly adjusted can be verified with this graph.

### CITATION LIST

### PATENT LITERATURE

Japanese Unexamined Patent Application Publication (Translation of PCT Application) No. 2009-531883

### SUMMARY

However, the following problems are encountered with the conventional hearing aid adjustment device discussed above.

### TECHNICAL PROBLEM

With the hearing aid adjustment device disclosed in the above-mentioned publication, the peak indicator shown on the display screen is considered to be the maximum sound level for audio that is continuous for several seconds. The phrase "audio that is continuous for several seconds" here means audio having a sentence-level length. Thus, with the adjustment method of Patent Literature 1, the hearing aid user can verify whether or not he has heard an entire sentence, but cannot correctly verify whether or not he has heard the words that make up the sentence or the sounds that make up the words. As a result, the hearing aid cannot be adjusted to a high level of user satisfaction.

It is an object of the present invention to provide a hearing aid adjustment device with which it is possible to verify the degree of hearing in word or sound (segment) units in hearing aid adjustment, and a hearing aid can be adjusted to a high level of satisfaction on the part of the hearing aid user.

### SOLUTION TO PROBLEM

The hearing aid adjustment device pertaining to the present invention is connected to a hearing aid and is used to adjust the hearing aid by using reference sounds, with said hearing aid adjustment device comprising a memory section, an output section, an input section, a controller, and a display section. The memory section stores reference sounds to which segment information has been assigned, as well as the desired volume range for the hearing aid user. The output section outputs a reference sound from the memory section to the hearing aid. The input section inputs hearing aid processed sounds with respect to reference sounds that have undergone hearing aid processing in the hearing aid. The controller calculates the volume of segment units included in a hearing aid processed sound on the basis of segment information stored in the memory section. The display section displays in segment units whether or not the volume calculated by the controller falls within the desired volume range for the hearing aid user as stored in the memory section.

### ADVANTAGEOUS EFFECTS

With the hearing aid adjustment device pertaining to the present invention, whether or not a hearing aid user can properly hear in segment units of reference sounds can be easily verified merely by looking at a displayed result, which means that a hearing aid can be adjusted more precisely than in the past. As a result, the hearing aid can be adjusted to a high level of satisfaction on the part of the hearing aid user.

### BRIEF DESCRIPTION OF DRAWINGS

FIG. 1 is a concept diagram of the hearing aid adjustment device pertaining to an embodiment of the present invention, and to peripheral devices thereof;
FIG. 2 is a control block diagram of the hearing aid adjustment device in FIG. 1;
FIG. 3 shows a sound source example of Japanese language stored in a sound source database of the hearing aid adjustment device in FIG. 1;
FIG. 4 shows a sound source example of English language stored in a sound source database of the hearing aid adjustment device in FIG. 1;
FIG. 5 shows an adjustment screen prior to the start of monitoring with the hearing aid adjustment device in FIG. 1;
FIG. 6 shows the initial state of the monitoring screen with the hearing aid adjustment device in FIG. 1; and
FIG. 7 shows an example of the screen outputted to the display section during evaluation sound source output with the hearing aid adjustment device in FIG. 1.

### DESCRIPTION OF EMBODIMENTS

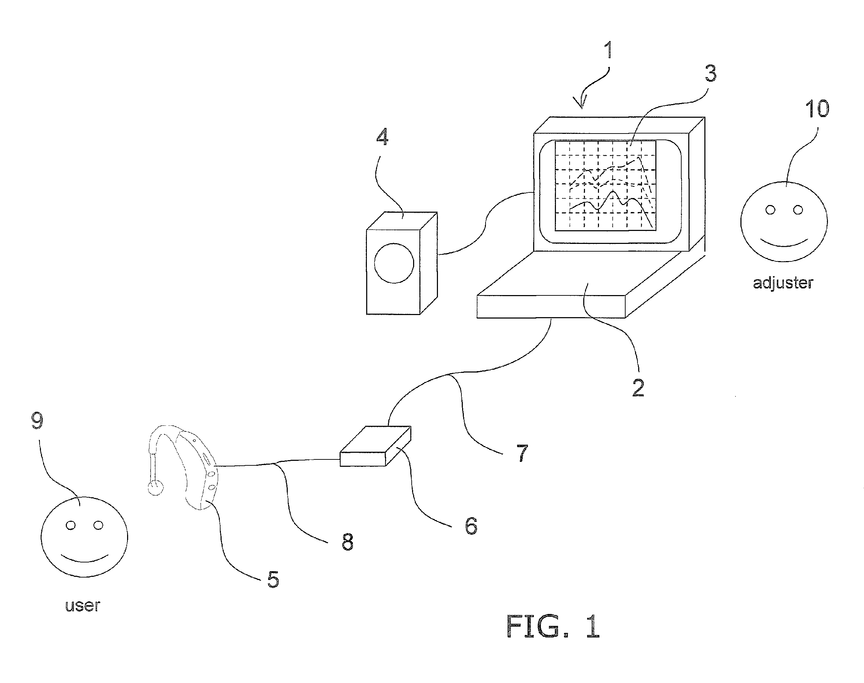

The hearing aid adjustment device pertaining to an embodiment of the present invention will now be described through reference to FIGS. 1 to 7.

As shown in FIG. 1, the hearing aid adjustment device 1 in this embodiment comprises an input section 2, a display section 3, and an output section 4. The hearing aid adjustment device 1 is connected to a hearing aid 5 to be adjusted, via a connector box 6 and wires 7 and 8.

The hearing aid 5 actually consists of two hearing aids for the left and right ears of a user 9, but just the hearing aid 5 for the right ear is shown as an example in FIG. 1.

An adjuster 10 sets up the hearing aid 5 by changing various parameters through the input section 2. Here, the output section 4 outputs an evaluation sound source for evaluating the adjustment.

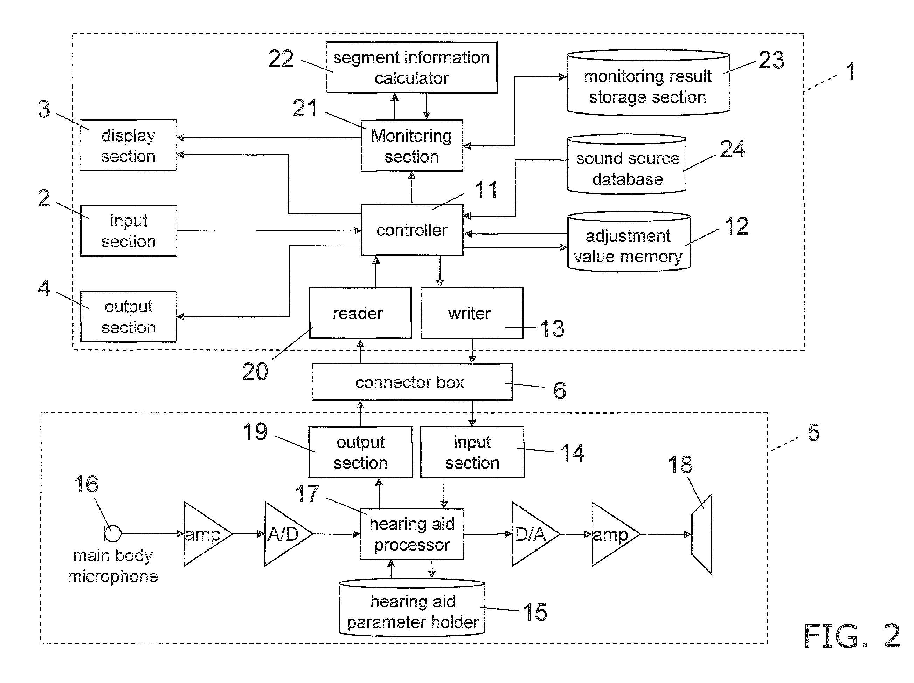

FIG. 2 shows the control blocks of the hearing aid adjustment device 1.

The adjustment values inputted and changed by the adjuster 10 through the input section 2 are stored in an adjustment value memory 12 and displayed on the display section 3. When the adjuster 10 inputs a command to write an adjustment value to the hearing aid 5, that adjustment value is written from a writer 13, via the connector box 6 and a hearing aid processor 17 and an input section 14 of the hearing aid 5, to a hearing aid parameter holder 15 of the hearing aid 5.

The hearing aid parameter holder 15 stores hearing aid parameters such as gain and compression decided according to the hearing ability of the user 9.

The hearing aid 5 uses the hearing aid processor 17 to process sound acquired from a main body microphone 16 on the basis of parameters held in the hearing aid parameter holder 15, after which it outputs the sound from a receiver 18, so that the user 9 is provided with sound that matches his sense of hearing and preferences.

Usually, the adjuster has the user 9 listen to a sound outputted from the hearing aid 5 that has been adjusted as above, and then fine tunes the hearing aid on the basis of this result.

With the hearing aid adjustment device 1 in this embodiment, a hearing aid adjustment method is performed in which this hearing aid processing is verified so that effective fine tuning will be possible. In this embodiment, to achieve this goal, an evaluation sound source (reference sounds) with meta information provided for each syllable is stored in a sound source database 24, and the state of hearing aid processing with respect to this evaluation sound source is monitored.

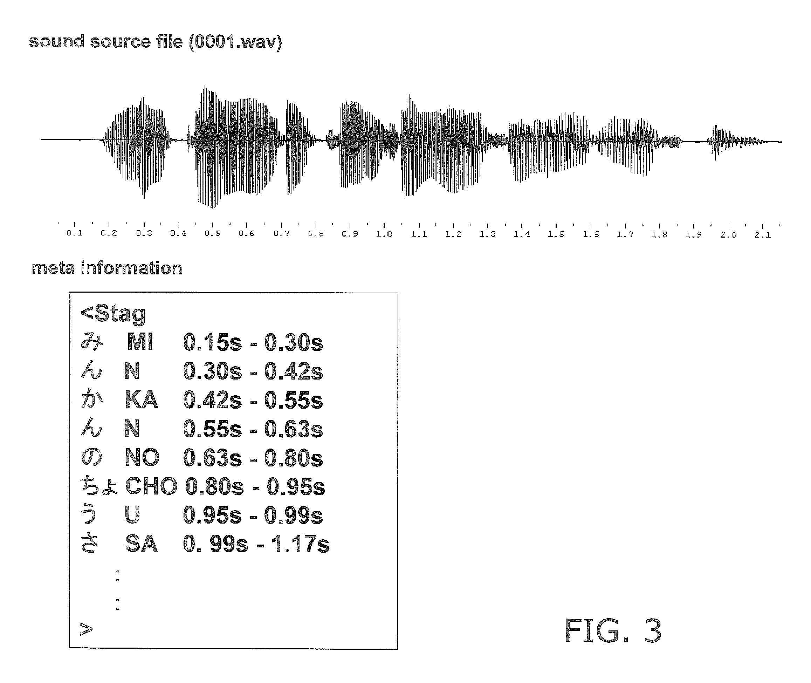

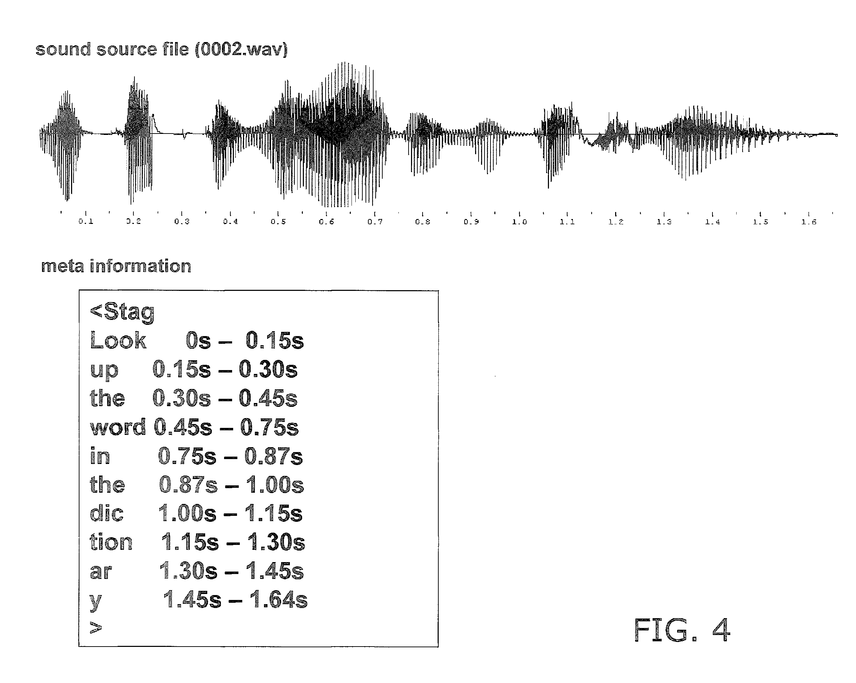

FIG. 3 shows an example of a Japanese sound source held in the sound source database 24, and FIG. 4 shows an example of an English sound source.

Here, a Wav file or another such sound source file, sound source syllable information and time information (segment time information) are assigned as meta information. In the Japanese sound source example shown in FIG. 3, the syllable information is given in both hiragana and Roman letters.

The adjuster uses the input section 2 to select a sound source to be provided to the user 9. The selected sound source is read by a controller 11 from the sound source database 24 and outputted from the output section 4. The sound source outputted from the output section 4 of the hearing aid adjustment device 1 is inputted through the main body microphone 16 to the hearing aid 5 and subjected to hearing aid processing by the hearing aid processor 17, after which it is outputted through the receiver 18 to the user 9.

In this embodiment, in order to verify the above-mentioned hearing aid processing, the sound inputted to the main body microphone 16 of the hearing aid 5 and the sound that has undergone hearing aid processing are returned by the hearing aid processor 17 to a reader 20 of the hearing aid adjustment device 1 via an output section 19 and the connector box 6.

These sounds are stored in a monitoring result storage section 23, a segment information calculator 22 refers to the meta information assigned to the sound source and calculates the average value and peak value of input sound pressure for each syllable and of sound pressure after hearing aid processing, and presents the results to the adjuster 10 on the display section 3 via a monitoring section 21.

The sounds inputted to the main body microphone 16 here are controlled so that they are returned to the hearing aid adjustment device 1, but the processing of these may be omitted. Specifically, if the "sounds inputted to the main body microphone 16" can be calculated from a parameter such as the distance between the hearing aid 5 and a speaker (output section 4) on the hearing aid adjustment device 1 side, then processing to return the sounds inputted to the main body microphone 16 back to the hearing aid adjustment device 1 need not be performed.

The operation of the hearing aid adjustment device 1 in this embodiment will now be described through reference to the drawings.

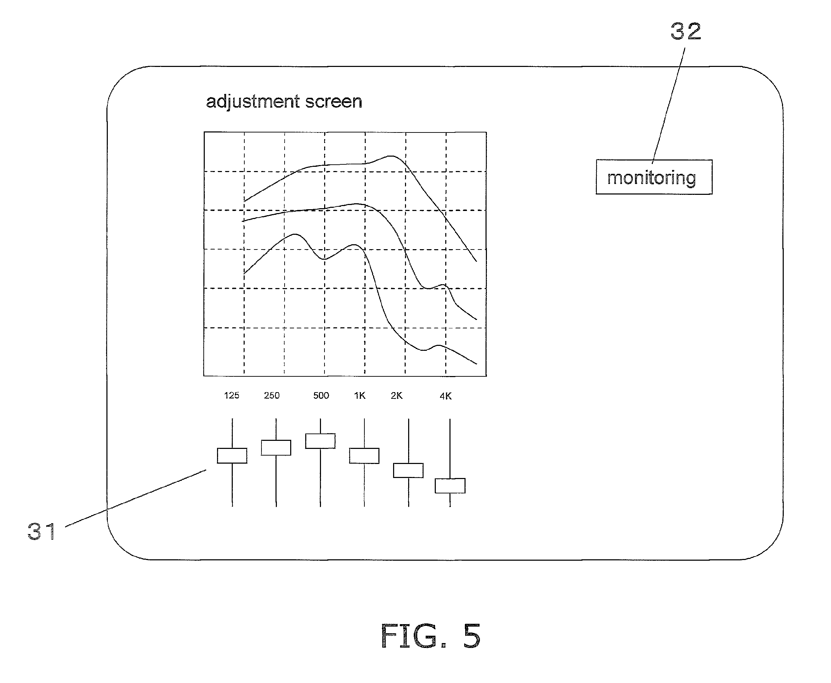

FIG. 5 shows an adjustment screen prior to the start of monitoring.

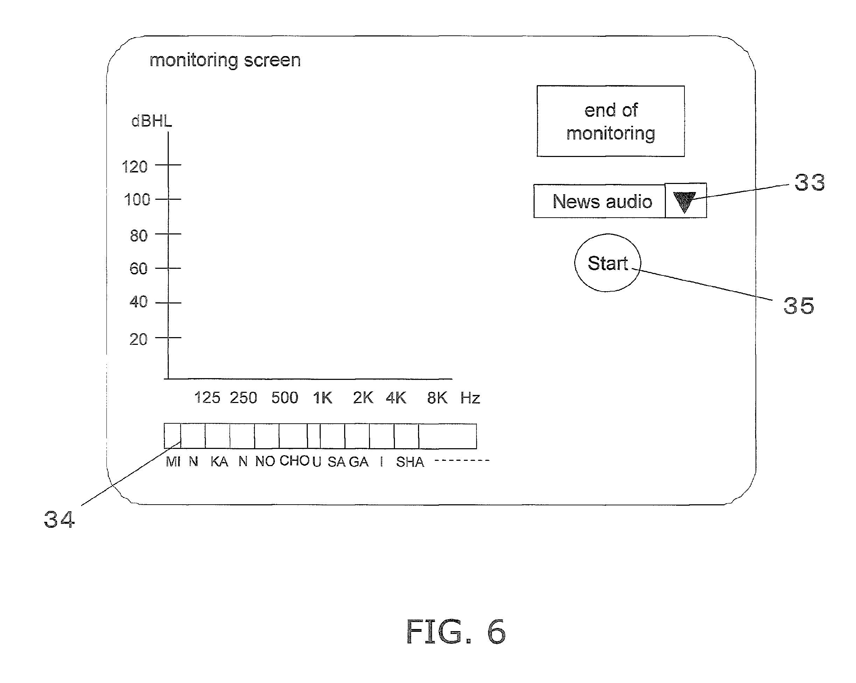

The adjuster 10 operates a slider 31 via the input section 2 to set the amplification for each frequency to suit the user 9. If a monitoring button 32 is pressed after a series of adjustments at each frequency, the adjustment screen shown in FIG. 5 changes to a specific monitoring screen (see FIG. 6). The three curves shown in FIG. 5 are, for example, the output sound pressure versus input sound pressures of 90 dB SPL, 60 dB SPL, and 40 dB SPL, starting from the top.

FIG. 6 shows the initial state of the monitoring screen.

The sound source to be used for evaluation is selected from a list of evaluation sound sources by using a pull-down menu 33. When a sound source is selected, syllable information and its segment time information are presented in a sound source information display region 34 on the basis of the meta information assigned to the sound source. With the sound source information display region 34 shown in FIG. 6, the size of a box indicates the length of each syllable.

Next, when a start button 35 is pressed by the adjuster 10, an evaluation sound source is outputted from the output section 4.

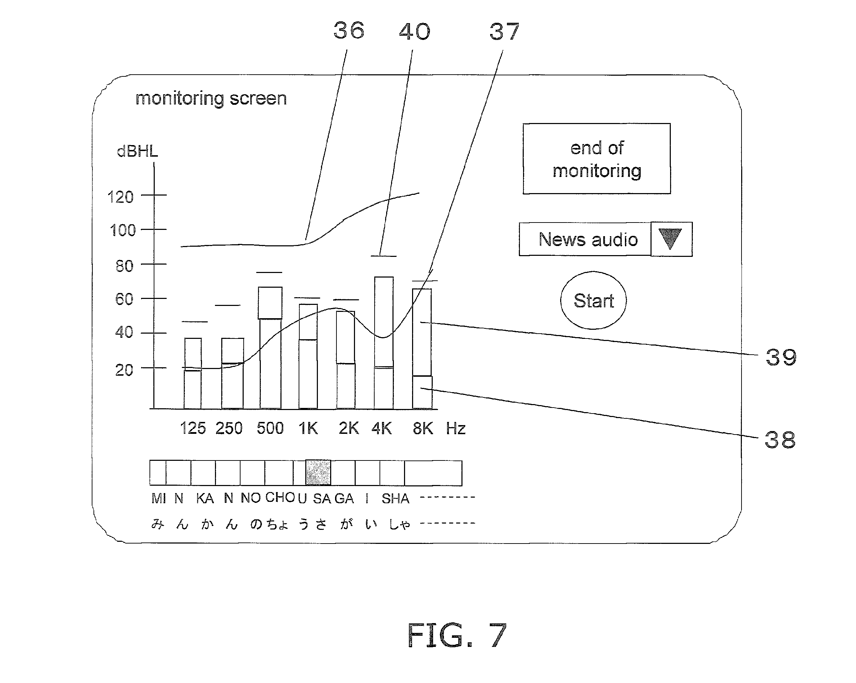

FIG. 7 shows an example of the screen outputted to the display section 3 during the output of an evaluation sound source.

When an evaluation sound source is outputted from the output section 4, that evaluation sound source is inputted through the main body microphone 16 to the hearing aid 5. After this, the evaluation sound source undergoes hearing aid processing by the hearing aid processor 17 on the basis of hearing aid parameters corresponding to the user 9.

The hearing aid processor 17 analyzes the strength at each frequency by fast Fourier transform (FFT) or other such processing on the sound inputted to the main body microphone 16, after which different amplification processing is performed for each frequency according to the hearing aid parameters, and processing to return to a waveform is performed by inverse discrete Fourier transform (IDFT). The strength thus found at each frequency (spectrum information, volume) is monitored on the screen.

More specifically, the uncomfortable level (UCL) 36 and hearing threshold (HTL) 37 of the user 9 are displayed on the screen, and the average value 38 of spectrum information prior to hearing aid processing for each segment time found from spectrum information prior to hearing aid processing, and the average value 39 of spectrum information for each segment time found from spectrum information after hearing aid processing are also displayed. Furthermore, the peak value 40 for each segment time corresponding to a syllable is displayed. The UCL 36 and HTL 37 set for each user 9 here are stored in the adjustment value memory (memory section) 12.

In this embodiment, whether or not the sound has been amplified to a level that the user can hear can be verified after hearing aid processing by confirming that the average value and peak value for each segment time are values between the uncomfortable level (UCL) and the hearing threshold (HTL) 37. That is, whether or not all of the syllables have been sufficiently amplified can be determined by using the average value and the peak value, and it can be confirmed that the peak value does not exceed the UCL 36 from the plosives and so forth that may be included in each syllable (each segment time).

In the example shown in FIG. 7, there is a characteristic at input frequencies of 500 Hz and 1 kHz, but it can be seen that of these two frequencies, at 1 kHz that sound can only be amplified to an extent that it slightly exceeds the HTL 37. Thus, to improve the hearing of the Japanese syllable "sa," it can be seen that adjustment should be made so as to raise the degree of amplification of the 1 kHz frequency portion.

With prior art, real-time spectrum information and the peak value for the past few seconds were displayed as the above-mentioned spectrum information. However, to confirm whether or not words can be heard, it is necessary not only to see information such as the peak value for the past few seconds, but also to determine whether or not hearing aid processing is suitable in units of phonemes (the smallest unit of language) or syllables.

In view of this, with the hearing aid adjustment device 1 in this embodiment, time information is acquired for each syllable from meta information assigned to an evaluation sound source. The segment information calculator 22 calculates the average value 39 and the peak value 40 on the basis of time information for each syllable from spectrum information before and after hearing aid processing obtained via the output section 19, the connector box 6, and the reader 20 from the hearing aid processor 17 on the hearing aid 5 side. This information is monitored on the display section 3. FIG. 7 shows an example of performing hearing aid processing again on the Japanese syllable "sa."

Furthermore, with the hearing aid adjustment device 1 of this embodiment, the configuration may be such that the above-mentioned result is stored in the monitoring result storage section 23, and the stored result can be rechecked after the end of reproduction of the evaluation sound source.

More specifically, the configuration may be such that each syllable can be selected with a pointing device or the like in the sound source information display region 34, and the average value or peak value in the evaluation of the selected syllable may be presented by the method shown in FIGS. 6 and 7.

### Other Embodiments

An embodiment of the present invention was described above, but the present invention is not limited to the above embodiment, and various modifications are possible without departing from the gist of the invention.

### (A)

In the above embodiment, an example was given of a case of monitoring hearing aid processing on the basis of segment information in syllable units, but the present invention is not limited to this. For example, syllable units are considered preferable for improving the accuracy of hearing language, but the same effect as in the above embodiment can also be obtained when using phoneme units, which are shorter than syllables, or when using word units, which are longer than syllables.

### (B)

In the above embodiment, an example was described of using an average value and a peak value to perform monitoring, but the present invention is not limited to this. For example, the same effect as in the above embodiment can also be obtained when monitoring with an average value and/or a peak value, although the accuracy may suffer.

### INDUSTRIAL APPLICABILITY

The hearing aid adjustment device of the present invention has the effect of being able to verify the extent of hearing in word or sound (segment) units in performing hearing aid adjustment, and to perform hearing aid adjustment to a high level of satisfaction on the part of the hearing aid user, and as such can be widely applied to hearing aids in general, regardless of whether they are for one or both ears.

### REFERENCE SIGNS LIST

- 1: hearing aid adjustment device
- 2: input section
- 3: display section
- 4: sound output section (output section)
- 5: hearing aid
- 6: connector box
- 7, 8: wire
- 9: user (hearing aid user)
- 10: adjuster
- 11: controller
- 12: adjustment value memory (memory section)
- 13: writer
- 14: input section
- 15: hearing aid parameter holder
- 16: main body microphone
- 17: hearing aid processor
- 18: receiver
- 19: output section
- 20: reader
- 21: monitoring section
- 22: segment information calculator
- 23: monitoring result storage section
- 24: sound source database (memory section)
- 31: slider
- 32: monitoring button
- 33: pull-down menu
- 34: sound source information display region
- 35: start button
- 36: uncomfortable level (UCL)
- 37: hearing threshold (HTL)
- 38, 39: average value
- 40: peak value

## Claims

1. A hearing aid adjustment device, which is connected to a hearing aid and is used to adjust the hearing aid by using reference sounds, said hearing aid adjustment device comprising:
a memory section that stores the reference sounds to which segment information has been assigned, and the desired volume range for the hearing aid user;
an output section that outputs the reference sounds from the memory section to the hearing aid;
an input section to which hearing aid processed sounds with respect to reference sounds that have undergone hearing aid processing in the hearing aid are inputted;
a controller that calculates the volume of segment units included in a hearing aid processed sound on the basis of the segment information stored in the memory section; and
a display section that displays in segment units whether or not the volume calculated by the controller falls within the desired volume range for the hearing aid user as stored in the memory section.

2. The hearing aid adjustment device according to Claim 1, wherein
the segment units include syllable units, phoneme units, and word units.

3. The hearing aid adjustment device according to Claim 1 or 2, wherein
the desired volume range is set so that the lower limit is the hearing threshold of the hearing user, and the upper limit is the uncomfortable threshold for the hearing aid user.

4. The hearing aid adjustment device according to any of Claims 1 to 3, wherein
the display section displays a comparison of the average value and peak value for the volume of the segment units included in the hearing aid processed sounds, with respect to the desired volume range.
